Europäisches Patentamt

European Patent Office

Office européen des brevets

(11) Publication number: **0 438 591 B1**

# EUROPEAN PATENT SPECIFICATION

(45) Date of publication of patent specification: **10.08.94**  (51) Int. Cl.[5]: **C12P 13/22**

(21) Application number: **88908987.6**

(22) Date of filing: **12.10.88**

(86) International application number:
**PCT/JP88/01036**

(87) International publication number:
**WO 89/03428 (20.04.89 89/09)**

(54) **PROCESS FOR PRODUCING L-TRYPTOPHANE.**

(30) Priority: **12.10.87 JP 254728/87**

(43) Date of publication of application:
**31.07.91 Bulletin 91/31**

(45) Publication of the grant of the patent:
**10.08.94 Bulletin 94/32**

(84) Designated Contracting States:
**AT BE CH DE FR GB IT LI NL**

(56) References cited:
**JP-A- 565 098**
**JP-A- 5 816 692**

**CHEMICAL ABSTRACTS, vol. 102, no. 25, 24th June 1985, page 187, abstract no. 216308d, Columbus, Ohio, US; Mitsubishi Petrochemical Co., Ltd., "Plasmid pMTY-1"; & JP-A-60 02 189**

(73) Proprietor: **MITSUI TOATSU CHEMICALS, Inc.**
**2-5 Kasumigaseki 3-chome**
**Chiyoda-Ku Tokyo 100 (JP)**

(72) Inventor: **OGAWA, S. 3-3-2, Mitsui Toatsu Iijima Ap. 2882**
**Iijima-cho**
**Sakae-ku, Yokohama-shi**
**Kanagawa 244 (JP)**
Inventor: **IGUCHI, Seiya**
**4-21-7, Kamirenjyaku**
**Mitaka-shi**
**Tokyo 181 (JP)**
Inventor: **MORITA, Satoshi**
**7-13-9, Kurihama**
**Yokosuka-shi**
**Kanagawa 239 (JP)**
Inventor: **KUWAMOTO, Hideharu**
**172, Yamanouchi**
**Kamakura-shi**
**Kanagawa 247 (JP)**

CHEMICAL ABSTRACTS, vol. 82, no. 9, 3rd March 1975, page 448, abstract no. 56064j, Columbus, Ohio, US; I. CHIBATA et al, "L-Tryptophan production by immobilized microbe"; & JP-A-74 81 591

PATENT ABSTRACTS OF JAPAN, vol. 12, no. 270 (C-515)[3117], 27th July 1988; & JP-A-63 49 091

AGRICULTURAL AND BIOLOGICAL CHEMISTRY, vol. 46,no. 11, November 1982, pages 2711-2718, JP; SHIN-ICHI SUGIMOTO et al, "Tryptophan synthase and production of L-tryptophan in regulatory mutants"

(74) Representative: Hayward, Denis Edward Peter et al
Lloyd Wise, Tregear & Co.
Norman House
105-109 Strand
London WC2R 0AE (GB)

EP 0 438 591 B1

**Description**

This invention relates to a process for producing L-tryptophan; more particularly, this invention relates to a process for continuously producing L-tryptophan utilizing an immobilized enzyme source.

L-tryptophan is a kind of amino acid, and is used in medical field as a component of transfusion liquid. Further, the use of L-tryptophan as an additive of cattle feed is now being developed, and the wide variety of uses thereof is expected.

Conventionally, L-tryptophan is usually manufactured by an enzyme reaction utilizing an enzyme such as tryptophan synthase and tryptophanase. These processes which utilize an immobilized enzyme source are drawing attention because the separation of L-tryptophan from the immobilized enzyme source and purification thereof are easy.

When L-tryptophan is industrially produced using an immobilized enzyme source, although a batch process can be employed in small scale production, a continuous process which is advantageous in various respects is demanded in large scale production.

The success of a continuous process depends on whether the lifetime of the activity of the immobilized enzyme source can satisfactorily be prolonged or not. One can try to prolong the lifetime of the activity of the immobilised enzyme source by improving the immobilised enzyme source per se and/or by the appropriate selection of the reaction conditions.

Although an improvement of the immobilised enzyme source or the enzyme per se has been studied by using the genetic recombination technique and the like, satisfactory results have not yet been obtained. Further, even if the immobilised enzyme source or the enzyme per se is improved, the prolongation of the lifetime of the activity largely depends on the appropriate selection of the reaction conditions, which takes a long time.

Heretofore, the prolongation of the lifetime of the immobilised enzyme has not yet been attained.

Therefore, an object of the present invention is to provide a process of producing L-tryptophan from L-serine and indole, in which the lifetime of the activity of the immobilised enzyme source is prolonged.

According to the invention there is provided a process of continuously producing L-tryptophan by reacting indole and L-serine using an immobilised enzyme source, characterised in that at any point of time the molar number of L-serine in the reaction mixture is greater than the total of the molar numbers of indole and L-tryptophan in the reaction mixture.

By the process of the present invention, the lifetime of the immobilised enzyme source can be prolonged irrespective of the amount of the accumulated L-tryptophan, so that L-tryptophan can be continuously produced from indole and L-serine for a long time. Further, the concentration of the produced L-tryptophan can be made higher than in the conventional processes.

As used herein, the term "immobilised enzyme source" means immobilised tryptophan synthase, tryptophanase or immobilised microorganism which produces at least one of these enzymes. Known tryptophan synthase-producing microorganisms include Escherichia coli MT-10232 (FERM BP-19), Escherichia coli MT-10242 (FERM BP-20) and Neurospora crassa (ATCC 14692). Known tryptophanase-producing microorganisms include Proteus bulgaris (IFO 3167), Escherichia coli (IAM 1268), Aerobacter aerogenes (IFO 12019) and Klebsiella penumoniae (ATCC 8724). The method of producing the immobilized enzyme source, that is, the method of immobilizing the enzyme or the microorganism is well-known in the art. For example, the enzyme or the microorganism can be immobilized in an alginate such as calcium alginate and aluminium alginate, or in a carrier such as carrageenan by a well-known conventional method.

In the process of the present invention, the molar number of L-serine in the reaction mixture is greater than the total of the molar numbers of indole and L-tryptophan. Although the effect of the present invention may be obtained even when the molar number of L-serine is slightly greater than the total of the molar numbers of indole and L-tryptophan, in cases where the concentration of the produced L-tryptophan is high, for example, in the case where L-tryptophan is continuously produced by using a multiple reactors or in the case where L-tryptophan is continuously produced by circulating the reaction mixture in a reactor, to sufficiently prolong the lifetime of the immobilized enzyme source so as to be suited as an industrial process, the molar number of L-serine in the reaction mixture is preferably 1.2 times the total molar number of the indole and L-tryptophan. Further, since indole more severely inhibits the enzyme activity than L-tryptophan, the molar number or L-serine in the reaction mixture is preferably 1.5 times greater than the molar number of indole alone. Even if the molar number of L-serine is much greater than the total molar number of indole and L-tryptophan, the effect of the present invention is not adversely affected. Thus, the excess molar number of L-serine may be appropriately selected so as to be suited for the recovery and recycle of the non-reacted L-serine. Usually, from the practical point of view, the molar number of L-serine is not more than 10 times the total of the molar numbers of indole and L-tryptophan. It should be noted that

3

racemic serine may be employed in the process of the present invention as long as the amount of L-serine in the racemic serine is in the range defined in the present invention. Further, needless to say, L-tryptophan need not be contained in the starting mixture.

Although not limited, the reaction may preferably be conducted at a temperature of 25 to 60°C. In order to promote the enzyme reaction rate and simultaneously to promote the solubility of L-tryptophan, the reaction is preferably conducted at a high temperature within the acceptable range.

Since L-tryptophan is an ampholyte and its solubility depends on the pH, it is preferred to adjust the pH higher or lower than its isoelectric point within the range acceptable for the enzyme activity so as to promote the concentration of the accumulated L-tryptophan. Thus, in many cases, the pH of the reaction mixture may be different from the optimum pH of the enzyme, and the lifetime of the immobilized enzyme source can be prolonged thereby. The pH of the reaction mixture is usually from 6 to 11, and preferably from 6.5 to 10.0.

In the process of the present invention, coenzymes such as pyridoxal phosphate may be added to the reaction mixture. Further, in cases where calcium alginate or aluminium alginate is used as the immobilizing carrier, calcium ion or aluminium ion may be present so as to prevent the disruption of the gel.

The reaction may be conducted by repeating batch process or by continuously flowing reaction mixture utilizing a tank reactor, or by continuously flowing reaction mixture utilizing a fixed bed reactor or a fluidized bed reactor. Further, these types of reactor can be combined. For example, feed reaction mixture containing indole and L-tryptophan as well as L-serine in excess of the total molar number thereof, and optionally the above-mentioned component for promoting the enzyme activity may be continuously supplied to a reactor at a prescribed flow rate and a temperature, and the reaction product mixture containing the L-tryptophan newly produced in the reactor may be continuously withdrawn from the reactor at the same flow rate as supplying the feed reaction mixture. In the especially preferred mode of the invention, as is shown in Example 3 described below, the reaction is carried out in a plurality of serially connected stirred tank reactors and the condition of the present invention is attained in each reaction bath. In this process, to the second or a subsequent reactor, reaction product mixture from the previous reactor is supplied. In this case, if the condition of the present invention becomes unsatisfied in any reactor, additional L-serine is supplied to the reactor in addition to the reaction product mixture from the previous reactor.

By the process of the present invention, it is possible to continue the production of L-tryptophan from indole and L-serine for as long as 200 hours irrespective of the L-tryptophan dissolved in the reaction mixture, and thus to prolong the lifetime of the immobilized enzyme source considerably. Further, by the process of the present invention, the concentration of L-tryptophan can be made higher than the conventional processes. For example, by continuously producing L-tryptophan using a plurality of reactors as described later or by continuously producing L-tryptophan by circulating the reaction mixture in a single reactor, an L-tryptophan concentration of 10 g/l or more may be attained, so that it is especially advantageous for the industrial production of L-tryptophan.

The present invention will now be described in more detail based on examples thereof.

Example 1

Preparation of Immobilized Enzyme Source

A tryptophan synthase-producing bacterium, Escherichia coli MT-10232 (FERM BP-19) was innoculated to 100 ml of a culture medium with a composition shown in Table 1, which is contained in a 500 ml flask and the resulting culture medium was incubated for 24 hours. Two hundred milliliters (two flasks) of the culture was innoculated to 15 litres of a culture medium with the composition shown in Table 2 contained in a 30 litre jar fermenter and the resulting culture medium was incubated at 35°C, pH6.8 (adjusted with 28% aqueous ammonia) for 30 hours.

Table 1

| Composition of Culture Medium | |
| --- | --- |
| Ehrlich's Meat Extract | 10 g |
| Polypeptone | 10 g |
| NaCl | 5 g |
| Dissolved in 1 liter of Distilled Water (pH 6.8) | |

Table 2

| Composition of Growth Medium | |
| --- | --- |
| Glucose | 10 g |
| $(NH_4)_2 SO_4$ | 1.5 g |
| $MgSO_4\ 7H_2O$ | 1 g |
| Polypeptone | 0.5 g |
| Yeast Extract | 0.5 g |
| L-tryptophan | 0.15 g |
| Surfactant (Adecanol LG-805, manufactured by Asahi Denka Co., Ltd.) Dissolved in 1 litre of distilled water (pH 6.8) | |

After completion of the culture, the culture medium was centrifuged to obtain 600 g wet weight of bacterial cells. The bacterial cells were placed in a sealed container and stored in a refrigerator at 4°C, and were used for preparing the immobilized enzyme source.

One part of the above-described wet bacterial cells and one part of physiological saline were mixed with stirring. On the other hand, 7.76 parts by weight of distilled water and 0.24 part by weight of sodium alginate (NSPLL manufactured by Kibun Food Chemifa, Co., Ltd.) were mixed with stirring and pH thereof was adjusted to 8.5 with sodium hydroxide.

Two parts of the suspension of the cells and 8 parts of the sodium alginate solution were mixed with stirring and the resulting mixture was fed into a syringe and was dropped into a gelling solution from the tip of a needle with an inner diameter of 0.5 mm to 0.8 mm.

The gelling solution was 0.5 M aqueous calcium chloride dihydrate solution of which pH was adjusted to 8.5 with 6 N potassium hydroxide solution, kept at 10°C,and was used in the amount of 10 parts.

The particles formed by dropping the mixture into the gelling solution was matured under stirring in the gelling solution for about 1 hour to obtain immobilized enzyme source.

Synthesis Reaction of L-tryptophan

Four solutions A, B, C and D, having the same composition except for the L-serine concentration as shown in Table 3 were prepared and the pH was adjusted to 8.5. One hundred millilitres of the each solution and 50 ml of the above-described immobilized bacterial cells were placed in a 500 ml glass reactor with a stirrer, and the reactor was placed in a warm water bath to keep the temperature of the reactor at 35°C. The solution with the same composition as the solution placed in each of the reactor was continuously fed at a flow rate of 50 ml and the reaction mixture was continuously withdrawn from the reactor at the same flow rate. Aliquotes of the reaction mixture were sampled at a fixed time interval, and the concentrations of L-tryptophan, remaining indole and remaining L-serine were determined using high performance liquid chromatography. The yield of the L-tryptophan newly produced in the reactor with respect to the supplied indole was determined, and the duration in which this yield is reduced to 50%, i.e., the half life of the enzyme activity was determined, which is shown in Table 3 as the lifetime of the enzyme.

Table 3

| Concentration of Component ( g/ℓ ) | Solution A | Solution B | Solution C | Solution D |
|---|---|---|---|---|
| Indole | 2.0 | 2.0 | 2.0 | 2.0 |
| L-tryptophane | 10.0 | 10.0 | 10.0 | 10.0 |
| Calcium Chloride Dihydrate | 1.5 | 1.5 | 1.5 | 1.5 |
| Pyridoxal Phosphate | 0.01 | 0.01 | 0.01 | 0.01 |
| L-serine | 6.94 | 8.33 | 13.9 | 27.8 |
| Molar Ratio of L-serin to Total of Indole and L-tryptophane | 1.0 | 1.2 | 2.0 | 4.0 |
| Lifetime (hours) | 180.0 | 625.0 | 892.0 | 1074.0 |

Example 2

The same continuous reaction as in Example 1 was repeated except that the composition of the feed reaction mixture was 30 g/l of L-tryptophan, 2 g/l of indole, 2.5 g/l of calcium chloride dihydrate, 10 mg/l of pyridoxal phosphate and 70 g/l of L-serine which is about 4 times the total of the molar numbers of indole and L-tryptophan, the pH of the reaction mixture was 10.0 and the reaction temperature was 40°C. The time in which the yield of L-tryptophan is reduced to 50% was 650 hours.

Example 3

In this Example, the preparation method of the immobilized enzyme source, the type of the reactor, the amount of the mixture in the reactor and the amount of the immobilized enzyme used were the same as in Example 1. However, in this Example, 4 reactors were serially connected via pipings and mixture from the previous reactor alone was fed to the subsequent reactor.

The reaction mixture with a composition shown in Table 4 was supplied to the first reactor at a rate of 48 ml/hour, and indole solution in methanol with a concentration of 62.5 g/l was added dropwise to the first to fourth reactors at a rate of 2 ml/hour. Further, to the third reactor, powder of L-serine was continuously added at a rate of 0.655 g/hour to conduct the synthesis reaction of L-tryptophan. Further, for the purpose of comparison, the same process was carried out except that L-serine is not added to the third reactor. In this case, although the reaction in the third reactor was carried out in the conditions defined in the present invention, in the fourth reactor, the amount of L-serine was smaller than the lower limit defined in the present invention.

Table 4

| | |
|---|---|
| L-serine | 11.7 g/l |
| pyridoxal phosphate | 0.01 g/l |
| calcium chloride dihydrate | 1.5 g/l |
| pH | 8.5 |

The lifetime of the enzyme in each reactor is shown in Table 5. Further, in Table 5, the molar ratio of L-serine to indole and the molar ratio of L-serine to the total of indole and L-tryptophane at the inlet of each reactor are also shown in Table 5.

Table 5

| | First Step | Second Step | Third Step | Fourth Step | Third Step (Comparative Example) | Fourth Step (Comparative Example) |
|---|---|---|---|---|---|---|
| Amount of Fed L-tryptophane (g/hr) | 0 | 0.214 | 0.427 | 0.641 | 0.427 | 0.641 |
| Amount of Fed Indole (g/hr) | 0.125 | 0.125 | 0.125 | 0.125 | 0.125 | 0.125 |
| Amount of Fed L-serine (g/hr) | 0.561 | 0.451 | 0.996 | 0.886 | 0.341 | 0.231 |
| Molar Ratio of L-serine/indole | 5.0 | 4.0 | 8.9 | 7.9 | 3.0 | 2.1 |
| Molar Ratio of L-serine/indole + L-tryptophane | 5.0 | 2.03 | 3.0 | 2.0 | 1.03 | 0.52 |
| Lifetime of Immobilized Enzyme (hr) | 1100 | 920 | 730 | 620 | 210 | 110 |

If the reaction is conducted using the first reactor only, even if the L-serine is present in excess, the maximum loading amount of indole in the feed reaction mixture is 0.125 g/h, and so the maximum production of L-tryptophan in the reaction mixture is 0.2179 g/h and its concentration is 4.36 g/l. These are not satisfactory for an industrial process.

As shown in this Example, if the reaction is continuously conducted in the conditions defined in the present invention (i.e., additional L-serine is added to the third reactor), the lifetime of the immobilized cells

7

can be greatly prolonged. On the other hand, if the additional L-serine is not added to the third reactor, the molar ratio of L-serine to indole and L-tryptophan is outside the range defined in the present invention, so that the lifetime of the immobilized cells cannot be satisfactorily prolonged.

The process of the present invention can be applied to the industrial continuous process of L-tryptophan which is useful as a component of transfusion liquid or an additive of cattle feed.

**Claims**

1.  A process of continuously producing L-tryptophan by reacting indole and L-serine using an immobilised enzyme source, characterised in that at any point of time the molar number of L-serine in the reaction mixture is greater than the total of the molar numbers of indole and L-tryptophan in the reaction mixture.

2.  A process as claimed in Claim 1 characterised in that the molar number of L-serine is not less than 1.2 times the total of the molar numbers of indole and L-tryptophan, and is not less than 1.5 times the molar number of indole.

3.  A process as claimed in Claim 1 characterised in that the reaction is conducted in a plurality of reactors, the condition for the molar number of L-serine set forth in Claim 1 is satisfied in each reactor, and the reaction product mixture from a previous reactor is added to a second or subsequent reactor and, if necessary for satisfying the condition for the molar number of L-serine set forth in Claim 1, L-serine is added to that second or subsequent reactor.

**Patentansprüche**

1.  Verfahren zur kontinuierlichen Herstellung von L-Tryptophan durch Reagieren von Indol und L-serin unter Verwendung einer unbeweglichen Enzymquelle, dadurch gekennzeichnet, daß die Molzahl von L-serin in dem Reaktionsgemisch zu jedem Zeitpunkt größer als die Molgesamtzahlen von Indol und L-Tryptophan in dem Reaktionsgemisch ist.

2.  Verfahren nach Anspruch 1, dadurch gekennzeichnet, daß die Molzahl von L-serin nicht weniger ist als 1,2 mal die Molgesamtzahlen von Indol und L-Tryptophan und nicht weniger ist als 1,5 mal die Molzahl von Indol.

3.  Verfahren nach Anspruch 1, dadurch gekennzeichnet, daß die Reaktion in einer Vielzahl von Reaktionsgefäßen erfolgt, wobei die in Anspruch 1 genannte Bedingung hinsichtlich der Molzahl von L-serin in jedem Reaktionsgefäß erfüllt wird und das erzeugte Reaktionsgemisch aus dem ersten Reaktionsgefäß in ein zweites oder folgendes Reaktionsgefäß gefüllt wird und, falls es für die in Anspruch 1 genannte Bedingung hinsichtlich der Molzahl von L-serin erforderlich ist, L-serin in das zweite oder folgende Reaktionsgefäß nachgefüllt wird.

**Revendications**

1.  Procédé de production continue de L-tryptophane en faisant réagir de l'indole et de la L-sérine on utilisant une source d'enzyme immobilisée, caractérisé en ce qu'en tout point quelconque dans le temps, le nombre molaire de la L-sérine dans le mélange de la réaction est supérieur au total des nombres molaires de l'indole et du L-tryptophane dans le mélange de la réaction.

2.  Procédé selon la revendication 1, caractérisé en ce que le nombre molaire de la L-sérine n'est pas inférieur à 1,2 fois le total des nombres molaires de l'indole et du L-tryptophane, et n'est pas inférieur à 1,5 fois le nombre molaire de l'indole.

3.  Procédé selon la revendication 1, caractérisé en cc que la réaction est conduite dans une pluralité de réacteurs, la condition pour le nombre molaire de la L-sérine indiqué dans la revendication 1 est satisfaite dans chaque réacteur, et le mélange du produit de la réaction provenant d'un réacteur précédent est ajouté à un second réacteur ou à un réacteur subséquent et, si nécessaire pour satisfaire la condition concernant le nombre molaire de la L-sérine indiquée dans la revendication 1, de la L-sérine est ajoutée à ce second réacteur ou au réacteur subséquent.